# EUROPÄISCHE PATENTANMELDUNG

(11) **EP 4 785 966 A1**
(43) Veröffentlichungstag der Anmeldung: **05.08.2026**
(21) Anmeldenummer: 25155753.4
(22) Anmeldetag: 04.02.2025
(51) Int. Cl.: A61L 9/12, A61L 9/14

(54) **ANORDNUNG UMFASSEND EINEN DIFFUSOR UND EINEN DEN DIFFUSOR UMGEBENDEN DESIGNRAHMEN**

(71) Anmelder: Albrecht Jung GmbH & Co. KG, 58579 Schalksmühle (DE)
(72) Erfinder: Rauch, Daniel, 42115 Wuppertal (DE)

(57) **Zusammenfassung**

Vorgestellt wird eine Anordnung 1 umfassend einen Diffusor DF und einen den Diffusor DF umgebenden Designrahmen D, wobei der Diffusor DF einen Tank T mit einem flüssigen Duftmittel DM und einen Zerstäuber zum Zerstäuben des flüssigen Duftmittels DM umfasst, wobei der Diffusor DF und der Designrahmen D auf einer Oberfläche O, insbesondere einer Wand, einer Fliese, einem Möbelstück oder einer Arbeitsplatte flächig angeordnet und mittels Festlegungsmitteln festgelegt sind, wobei der Zerstäuber ein manuelles Betätigungselement B zur Betätigung des Zerstäubers aufweist.

## Beschreibung

Die Erfindung betrifft eine Anordnung umfassend einen Diffusor und einen den Diffusor umgebenden Designrahmen, wobei der Diffusor einen Tank mit einem flüssigen Duftmittel und einen Zerstäuber zum Zerstäuben des flüssigen Duftmittels umfasst, wobei der Diffusor und der Designrahmen auf einer Oberfläche, insbesondere einer Wand, einer Fliese, einem Möbelstück oder einer Arbeitsplatte flächig angeordnet und mittels Festlegungsmitteln festgelegt sind, wobei der Zerstäuber ein manuelles Betätigungselement zur Betätigung des Zerstäubers aufweist.

Funktionsmodule, die dazu dienen, den Funktionsumfang von Installationsgeräten der Gebäudeinstallationstechnik zu erweitern, sind beispielsweise aus DE 10 2023 133 998 B3 oder DE 10 2023 110 987 B3 bekannt. Dabei handelt es sich typischerweise um Dimmer, Jalousieaktoren, Schaltaktoren oder dergleichen. Reizvoll dabei ist, dass die Hürde für die Nachrüstung weiterer Funktionen insofern abgesenkt ist, als dass keine vollständige Neuinstallation erforderlich ist, sondern die Funktionsmodule regelmäßig nachrüstbar sind.

Ein im Rahmen der Gebäudeinstallationstechnik eher selten bearbeitetes Gebiet betrifft Funktionsmodule, die dazu geeignet sind, die Raumluft zu beduften.

Bekannt sind Neuinstallationen, die in Installationsdosen installiert werden, beispielsweise aus EP 4 157 370 A1, und zur Raumluftverbesserung dienen. Diese sind insofern aufwendig, als dass sie die Installation einer Installationsdose mit entsprechender Verkabelung erfordern, so dass die Hürde für eine Nachrüstung hoch liegt, wobei regelmäßig ein Elektriker involviert wird.

Ebenfalls sind Duftdiffusoren bekannt, die via Plug-and-Play mittels Stecker in eine Steckdose, insbesondere in eine zylindrische Vertiefung mit zwei Steckdosenöffnungen, einsteckbar sind. Diese Lösungen sind jedoch weder ästhetisch noch kompakt, da sie ein Netzteil erfordern und bauraum ineffizient ausgeführt sind. Dadurch erhalten diese in eine Steckdose einsteckbare Duftdiffusoren eine ausladende Geometrie, so dass sie regelmäßig 10cm und mehr von der Steckdose abstehen. Damit entsteht das Problem, dass die Duftdiffusoren nicht nur wackelig anmuten, sondern auch eine Stolperfalle für Personen darstellen beziehungsweise durch Kollision leicht beschädigt werden können.

Ausgehend von diesem diskutierten Stand der Technik liegt der Erfindung die Aufgabe zugrunde, eine Anordnung mit einem Diffusor bereitzustellen, die nicht nur kompakt ist und sich in ein Installationsgeräteprogramm der Gebäudeinstallationstechnik einfügt, sondern darüber hinaus auch im Wege der Nachrüstung ohne das Herstellen einer neuen Installationsdose und jeglicher Verkabelung montierbar ist.

Gelöst wird diese Aufgabe durch eine Anordnung umfassend einen Diffusor und einen den Diffusor umgebenden Designrahmen, wobei der Diffusor einen Tank mit einem flüssigen Duftmittel und einen Zerstäuber zum Zerstäuben des flüssigen Duftmittels umfasst, wobei der Diffusor und der Designrahmen auf einer Oberfläche, insbesondere einer Wand, einer Fliese, einem Möbelstück oder einer Arbeitsplatte flächig angeordnet und mittels Festlegungsmitteln festgelegt sind, wobei der Zerstäuber ein manuelles Betätigungselement zur Betätigung des Zerstäubers aufweist.

Ein Vorteil der erfindungsgemäßen Anordnung besteht darin, dass auf geschickte Art und Weise ein Designrahmen eines Installationsgeräteprogramms der Gebäudeinstallationstechnik genutzt wird, um einen Diffusor zu umgeben. Dieses eröffnet nicht nur die optisch ansprechende Integration in ein Installationsgeräteprogramm, sondern verbessert zudem die Festlegung des Diffusors an einer Wand, einer Fliese, einem Möbelstück oder einer Arbeitsplatte.

Die Frage der Festlegung ist insofern relevant, als dass durch die erfindungsgemäße Anordnung vorteilhafterweise eine Festlegung an einer Installationsdose (wie sie im Rahmen der Gebäudeinstallationstechnik üblich ist) gerade vermieden wird. Mithin wird die Installationshürde herabgesetzt, da kein Elektriker erforderlich ist.

Die Festlegung des Diffusors und des Designrahmen auf einer Oberfläche erfolgt mittels Festlegungsmitteln, beispielsweise mittels Verrastung, Verschrauben oder Verkleben. Dieses umfasst insbesondere ein Verrastung, Verschrauben oder Verkleben einer optionalen Montageplatte an einer Oberfläche und eine Festlegung der erfindungsgemäßen Anordnung mittels Verrastung, Verschrauben oder Verkleben an der Montageplatte.

Dadurch dass der Diffusor und der Designrahmen auf der Oberfläche flächig angeordnet sind (der Ausdruck flächig im Sinne der Erfindung meint, dass der Diffusor eine Rückseite mit einer Fläche von mindestens 40 cm² aufweist, die zur Oberfläche weist), wird nicht nur eine installationsgerätetypische, besonders ästhetisch anmutende Kompaktheit ermöglicht, sondern im Gegensatz zu vorbekannten Einsteck-Lösungen, bei denen ein Diffusor mit Stecker in eine Steckdose eingesetzt - mithin nicht festgelegt - wird, ein Wackeln wirksam vermieden.

Im Sinne der Erfindung umfasst ein Zerstäuben eine ventilatorbasierte Diffusion, eine heizbasierte Verdampfung, eine Kaltluftzerstäubung oder eine Erzeugung eines Ultraschall-Nebels.

Gemäß einer außerordentlich bevorzugten Weiterbildung der erfindungsgemäßen Anordnung ist der Designrahmen ein Mehrfachrahmen mit mindestens einem ersten und einem zweiten Kompartiment. Dabei ist der Mehrfachrahmen an einer Installationsdose festgelegt und in das erste Kompartiment der Diffusor und in das zweite Kompartiment ein Installationsgerät der Gebäudeinstallationstechnik eingesetzt. Damit wird es auf intelligente Weise ermöglicht, die Festlegung der erfindungsgemäßen Anordnung besonders stark und mit wenig Aufwand auszubilden, indem eine benachbarte Installationsdose genutzt wird. Dabei kann die Festlegung insbesondere auch mittelbar erfolgen, beispielsweise über einen Halteadapter, der an einem Tragring an der Installationsdose festgelegt ist, wobei der Halteadapter nicht nur das in der Installationsdose befindliche Installationsgerät festlegt, sondern auch die erfindungsgemäße Anordnung.

Gemäß einer Weiterbildung der vorstehend genannten erfindungsgemäßen Anordnung ist das Installationsgerät der Gebäudeinstallationstechnik ein Schalter oder Taster, insbesondere zur Steuerung einer Leuchte neben einer Tür. Dadurch wird eine unkomplizierte, schnelle Betätigung ermöglicht, die dem typischen Nutzungsverhalten beim Betreten und Verlassen eines Raumes, insbesondere eines Toilettenraumes, entspricht:
Während des Prozesses des Lichtausschaltens (regelmäßig wenige Sekunden davor wird mit einer einfachen manuellen Betätigung des Betätigungselementes zur Aktivierung des Zerstäubers, ohne Positionswechsel der betätigenden Person komfortabel eine Zerstäubung des flüssigen Duftmittels erreicht).

In einer Weiterbildung der erfindungsgemäßen Anordnung weist der Diffusor Einstellmittel zur Einstellung der Diffusorleistung auf. Die Diffusorleistung ist ein Maß für die Freisetzung von Zerstäubung von Duftmittel. Eine solche Einstellung der Diffusorleistung kann beispielsweise erfolgen, indem das Einstellmittel als Schieber ausgeführt ist, der einen Austritt für zerstäubtes Duftmittel kontinuierlich öffnet beziehungsweise verschließt.

In einer Weiterbildung der erfindungsgemäßen Anordnung weist der Diffusor eine Erhebung im zentralen Bereich auf, die von seinem Rand beabstandet ist, so dass die Höhe des Diffusors vom Rand aus zunimmt. Hierdurch wird nicht nur das Tankvolumen vergrößert, sondern eine optisch schlanke Erscheinung gesichert.

Gemäß einer Weiterbildung der erfindungsgemäßen Anordnung beträgt die Breite und Höhe des Diffusors weniger als 9 cm und die Tiefe weniger als 6 cm. Hierdurch wird eine besondere Kompaktheit der erfindungsgemäßen Anordnung sichergestellt, obwohl sie gerade ohne unterputz angeordnete Installationsdose auskommt.

In einer Weiterbildung der erfindungsgemäßen Anordnung ist der Tank mechanisch reversibel an den Diffusor ankoppelbar, um das Befüllen oder Ersetzen (falls der Tank als Kartusche ausgeführt ist) des Tanks zu erleichtern. Zum Befüllen kann die erfindungsgemäße Anordnung auch um ein Abfüllwerkzeug, insbesondere einen Trichter, erweitert werden.

In einer Weiterbildung der erfindungsgemäßen Anordnung ist der Tank transparent ausgeführt, um eine optische Füllstandsanzeige zu ermöglichen.

Gemäß einer bevorzugten Weiterbildung der erfindungsgemäßen Anordnung, die sich chemisch besonders resistent gegenüber typischen Duftmitteln zeigt, ist der Tank aus Glas oder Keramik ausgeführt. Damit kann eine besonders hohe Konzentration des Duftmittels gewählt und eine kompakte Bauform gewährleistet werden, ohne dass der Tank chemisch angegriffen wird und seine Lebensdauer in Mitleidenschaft gezogen wird.

Nachfolgend ist die Erfindung unter Bezugnahme auf die beigefügten Figuren anhand dreier Ausführungsbeispiele beschrieben. Es zeigen:
- **Fig. 1:**: eine schematische Darstellung der erfindungsgemäßen Anordnung in einer perspektivischen Explosionsdarstellung in einer Vorderansicht (links) und in einer perspektivischen Darstellung in einer Vorderansicht (rechts),
- **Fig. 2:**: eine schematische Darstellung der erfindungsgemäßen Anordnung in einer perspektivischen Explosionsdarstellung in einer Rückansicht (links) und in einer perspektivischen Darstellung in einer Vorderansicht (rechts), jeweils mit einem benachbarten Installationsgerät und Mehrfachrahmen,
- **Fig. 3:**: eine schematische Frontdarstellung zweier erfindungsgemäßer Anordnungen mit einem beziehungsweise zwei benachbarten Installationsgeräten und entsprechenden Mehrfachrahmen und
- **Fig. 4:**: eine schematische Seitenansicht einer auf einer Oberfläche angeordneten erfindungsgemäßen Anordnung, wobei die Austrittsebene einen Winkelbereich W von 120° bis 180°, mit der Oberfläche bildet, die dafür sorgt, dass das Duftmittel nicht nur auf das Installationsgerät, sondern auch nicht auf die Oberfläche gelangt.

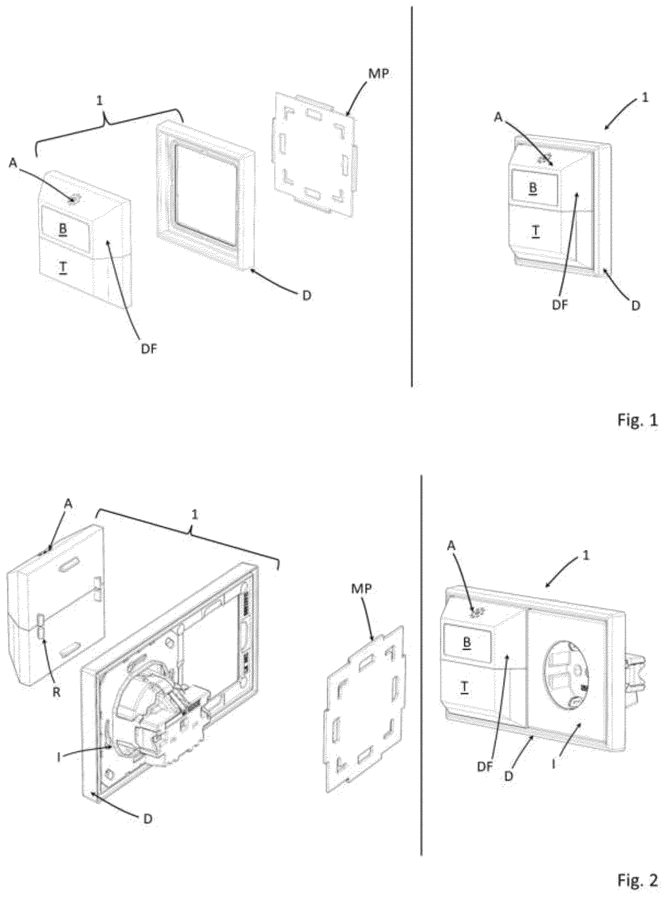

Figur 1 zeigt eine schematische Darstellung der erfindungsgemäßen Anordnung 1 in einer perspektivischen Explosionsdarstellung in einer Vorderansicht (links) und in einer perspektivischen Darstellung in einer Vorderansicht (rechts). Die erfindungsgemäße Anordnung 1 umfasst einen Diffusor DF und einen den Diffusor DF umgebenden Designrahmen D, wobei der Diffusor DF einen Tank T mit einem flüssigen Duftmittel DM und einen Zerstäuber zum Zerstäuben des flüssigen Duftmittels DM umfasst. Dabei ist der Diffusor DF und der Designrahmen D regelmäßig auf einer Oberfläche O, insbesondere einer Wand, einer Fliese, einem Möbelstück oder einer Arbeitsplatte flächig angeordnet und mittels Festlegungsmitteln festgelegt. Der Zerstäuber weist ein manuelles Betätigungselement B zur Betätigung des Zerstäubers auf, so dass eine elektrische Fachkraft zur Installation gerade nicht erforderlich ist, obwohl der optische Gesamteindruck eines elektrischen/elektronischen Installationsgerätes I geschaffen wird. Der dargestellte Diffusor DF weist ferner einen Austritt A auf, durch den zerstäubtes Duftmittel DM hindurchtritt, beispielsweise um einen Toilettenraum, einen Büroraum oder einen Wohnraum zu beduften. Zur einfachen Montage an einer Oberfläche O (in Figur 1 nicht dargestellt) wird der abgebildete Diffusor DF mittels Festlegungsmitteln festgelegt - dieses ist vorliegend beispielsweise durch ein Verschrauben oder Verkleben der Montageplatte MP an der Wand und anschließendes Verrasten des Diffusors DF an der Montageplatte MP möglich, wobei der Designrahmen D durch den Diffusor DF gehalten wird.

Figur 2 zeigt eine schematische Darstellung der erfindungsgemäßen Anordnung 1 in einer perspektivischen Explosionsdarstellung in einer Rückansicht (links) und in einer perspektivischen Darstellung in einer Vorderansicht (rechts), jeweils mit einem beziehungsweise zwei benachbarten Installationsgeräten I und einem Mehrfachrahmen. Dabei ist der Designrahmen D ein Mehrfachrahmen mit einem ersten und einem zweiten Kompartiment, wobei der Mehrfachrahmen an einer Installationsdose (nicht dargestellt) festgelegt ist, und in das erste Kompartiment der Diffusor DF und in das zweite Kompartiment ein Installationsgerät I der Gebäudeinstallationstechnik eingesetzt ist. Dadurch wird die optische Integration in ein Installationsgeräteprogramm nicht nur spürbar verbessert, sondern darüber hinaus auch auf effektive Weise sichergestellt, dass die erfindungsgemäße Anordnung 1 sogar ohne Verschrauben, also besonders einfach, festgelegt ist. Dieses wird beispielsweise erreicht, indem der Mehrfachrahmen in Wirkverbindung mit dem Diffusor DF und einer Montageplatte MP gebracht wird, wobei das an der Installationsdose festgelegte Installationsgerät I über den Mehrfachrahmen den Diffusor DF, der über Rastelemente R an dem Mehrfachrahmen festgelegt ist, an die Oberfläche O zieht beziehungsweise an dieser festlegt. Hierdurch wird eine vollständig reversible Festlegung realisiert. Derartige Rastelemente R können dabei entweder direkt in den Mehrfachrahmen eingreifen oder gekoppelt über die Montageplatte MP mit dem Mehrfachrahmen in mechanische Wirkverbindung gebracht werden. Alternativ kann eine solche, den Diffusor DF festlegende Wirkverbindung auch durch einen Halteadapter realisiert werden, der an dem ersten Installationsgerät I, beispielsweise einem Schalter oder Taster, festgelegt ist. Im rechten Bereich der Figuren 1 und 2 ist gezeigt, dass der Diffusor DF und der Tank T eine Erhebung H im zentralen Bereich aufweisen, die von seinem Rand beabstandet ist, so dass die Höhe des Diffusors und/oder des Tanks vom Rand aus zunimmt. Damit wird nicht nur ein kompakt anmutendes Design ermöglicht, sondern auch sichergestellt, dass mechanische Kollisionen nicht zu einem Abreißen des Diffusors führen, da eine seitlich angreifende Kraft abgelenkt wird. Ferner ist bevorzugt, wenn der Tank T transparent, insbesondere aus Glas, ausgeführt ist, um eine optische Füllstandsanzeige zu ermöglichen. Dabei zeichnet sich das Glas gleichzeitig als chemisch besonders resistent gegenüber hochkonzentrierten Duftmitteln DM aus, so dass die Lebensdauer erhöht und das Tankvolumen minimiert werden können, was positiv auf die Kompaktheit einzahlt.

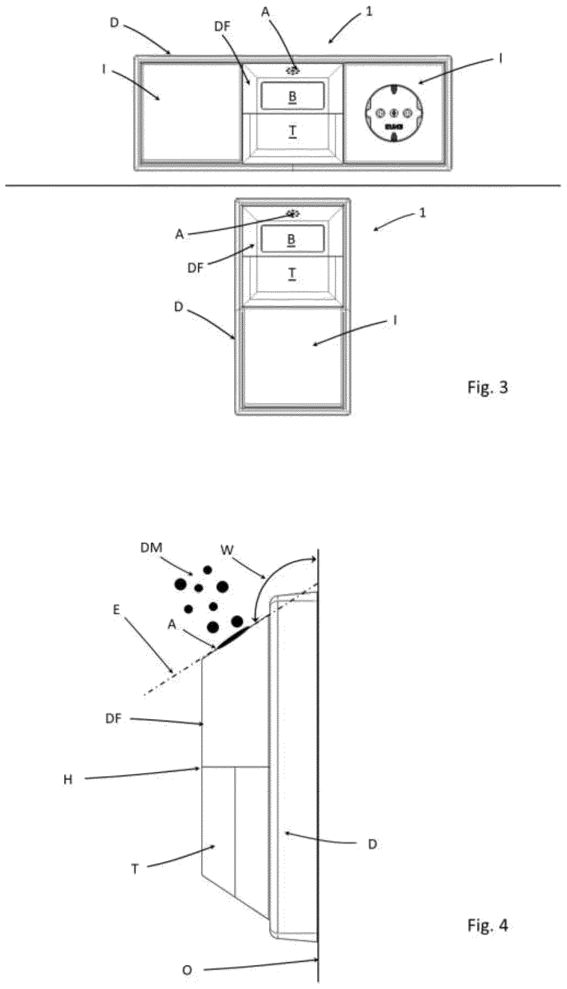

Figur 3 zeigt eine schematische Frontdarstellung zweier erfindungsgemäßer Anordnungen 1 mit einem beziehungsweise zwei benachbarten Installationsgeräten I und entsprechenden Mehrfachrahmen. In der oben dargestellten erfindungsgemäßen Anordnung 1 befindet sich der Diffusor DF zwischen zwei Installationsgeräten I, hier einer Steckdose und einem Schalter. Dadurch wird die Festlegung noch weiter vereinfacht, da der Mehrfachrahmen den Diffusor DF symmetrisch an die Installationsdosen der benachbarten Installationsgeräte I heranzieht. In der unten dargestellten erfindungsgemäßen Anordnung 1 befindet sich der Diffusor DF oberhalb eines Installationsgerätes I, hier eines Schalters oder Tasters, so dass zerstäubtes Duftmittel DM nicht auf das Installationsgerät I, insbesondere nicht in die Wippe des Schalters oder Tasters, gelangt, sondern der Austritt A, dessen Austrittsebene E bevorzugt einen Winkelbereich W von 100° bis 180°, insbesondere 120° bis 180°, mit der Oberfläche O bildet, dafür sorgt, dass das Duftmittel DM nicht nur nicht auf das Installationsgerät I sondern auch nicht auf die Oberfläche O gelangt (siehe Figur 4).

### Bezugszeichenliste

- 1: erfindungsgemäße Anordnung
- A: Austritt
- B: Betätigungselement
- D: Designrahmen
- DF: Diffusor
- DM: Duftmittel
- E: Austrittsebene
- H: Erhebung
- I: Installationsgerät
- MP: Montageplatte
- O: Oberfläche
- R: Rastelement
- T: Tank

## Patentansprüche

1. Anordnung (1) umfassend einen Diffusor (DF) und einen den Diffusor (DF) umgebenden Designrahmen (D), wobei der Diffusor (DF) einen Tank (T) mit einem flüssigen Duftmittel (DM) und einen Zerstäuber zum Zerstäuben des flüssigen Duftmittels (DM) umfasst, wobei der Diffusor und der Designrahmen (D) auf einer Oberfläche (O), insbesondere einer Wand, einer Fliese, einem Möbelstück oder einer Arbeitsplatte flächig angeordnet und mittels Festlegungsmitteln festgelegt sind, wobei der Zerstäuber ein manuelles Betätigungselement (B) zur Betätigung des Zerstäubers aufweist.

2. Anordnung (1) nach Anspruch 1, **dadurch gekennzeichnet, dass** der Designrahmen (D) ein Mehrfachrahmen mit mindestens einem ersten und einem zweiten Kompartiment ist, wobei der Mehrfachrahmen an einer Installationsdose festgelegt ist und in das erste Kompartiment der Diffusor (DF) und in das zweite Kompartiment ein Installationsgerät (I) der Gebäudeinstallationstechnik eingesetzt ist.

3. Anordnung (1) nach Anspruch 2, **dadurch gekennzeichnet, dass** das Installationsgerät (I) der Gebäudeinstallationstechnik ein Schalter oder Taster, insbesondere zur Steuerung einer Leuchte neben einer Tür, ist.

4. Anordnung (1) nach einem der Ansprüche 1 bis 3, **dadurch gekennzeichnet, dass** die Festlegungsmittel eine Verrastung, ein Verschrauben oder ein Verkleben umfassen.

5. Anordnung (1) nach einem der Ansprüche 1 bis 4, **dadurch gekennzeichnet, dass** der Diffusor (DF) Einstellmittel zur Einstellung der Diffusorleistung aufweist.

6. Anordnung (1) nach einem der Ansprüche 1 bis 5, **dadurch gekennzeichnet, dass** der Diffusor (DF) und/oder der Tank (T) eine Erhebung (H) im zentralen Bereich aufweist, die von seinem Rand beabstandet ist, so dass die Höhe des Diffusors (DF) und/oder des Tanks (T) vom Rand aus zunimmt.

7. Anordnung (1) nach einem der Ansprüche 1 bis 6, **dadurch gekennzeichnet, dass** die Breite und Höhe des Diffusors (DF) weniger als 9 cm und die Tiefe weniger als 6 cm beträgt.

8. Anordnung (1) nach einem der Ansprüche 1 bis 7, **dadurch gekennzeichnet, dass** der Tank (T) mechanisch reversibel an den Diffusor (DF) ankoppelbar ist.

9. Anordnung (1) nach einem der Ansprüche 1 bis 8, **dadurch gekennzeichnet, dass** der Tank (T) transparent ausgeführt ist, um eine optische Füllstandsanzeige zu ermöglichen.

10. Anordnung (1) nach einem der Ansprüche 1 bis 9, **dadurch gekennzeichnet, dass** der Tank (T) aus Glas oder Keramik ausgeführt ist.

## Geänderte Patentansprüche

### Geänderte Patentansprüche gemäss Regel 137(2) EPÜ.

1. Anordnung (1) umfassend einen Diffusor (DF) und einen den Diffusor (DF) umgebenden Designrahmen (D), wobei der Diffusor (DF) einen Tank (T) mit einem flüssigen Duftmittel (DM) und einen Zerstäuber zum Zerstäuben des flüssigen Duftmittels (DM) umfasst, wobei der Diffusor und der Designrahmen (D) auf einer Oberfläche (O), insbesondere einer Wand, einer Fliese, einem Möbelstück oder einer Arbeitsplatte flächig angeordnet und mittels Festlegungsmitteln festgelegt sind, wobei der Zerstäuber ein manuelles Betätigungselement (B) zur Betätigung des Zerstäubers aufweist, **dadurch gekennzeichnet, dass** der Designrahmen (D) ein Mehrfachrahmen mit mindestens einem ersten und einem zweiten Kompartiment ist, wobei der Mehrfachrahmen an einer Installationsdose festgelegt ist und in das erste Kompartiment der Diffusor (DF) und in das zweite Kompartiment ein Installationsgerät (I) der Gebäudeinstallationstechnik eingesetzt ist.

2. Anordnung (1) nach Anspruch 1, **dadurch gekennzeichnet, dass** das Installationsgerät (I) der Gebäudeinstallationstechnik ein Schalter oder Taster, insbesondere zur Steuerung einer Leuchte neben einer Tür, ist.

3. Anordnung (1) nach einem der Ansprüche 1 bis 2, **dadurch gekennzeichnet, dass** die Festlegungsmittel eine Verrastung, ein Verschrauben oder ein Verkleben umfassen.

4. Anordnung (1) nach einem der Ansprüche 1 bis 3, **dadurch gekennzeichnet, dass** der Diffusor (DF) Einstellmittel zur Einstellung der Diffusorleistung aufweist.

5. Anordnung (1) nach einem der Ansprüche 1 bis 4, **dadurch gekennzeichnet, dass** der Diffusor (DF) und/oder der Tank (T) eine Erhebung (H) im zentralen Bereich aufweist, die von seinem Rand beabstandet ist, so dass die Höhe des Diffusors (DF) und/oder des Tanks (T) vom Rand aus zunimmt.

6. Anordnung (1) nach einem der Ansprüche 1 bis 5, **dadurch gekennzeichnet, dass** die Breite und Höhe des Diffusors (DF) weniger als 9 cm und die Tiefe weniger als 6 cm beträgt.

7. Anordnung (1) nach einem der Ansprüche 1 bis 6, **dadurch gekennzeichnet, dass** der Tank (T) mechanisch reversibel an den Diffusor (DF) ankoppelbar ist.

8. Anordnung (1) nach einem der Ansprüche 1 bis 7, **dadurch gekennzeichnet, dass** der Tank (T) transparent ausgeführt ist, um eine optische Füllstandsanzeige zu ermöglichen.

9. Anordnung (1) nach einem der Ansprüche 1 bis 8, **dadurch gekennzeichnet, dass** der Tank (T) aus Glas oder Keramik ausgeführt ist.
